# EUROPEAN PATENT APPLICATION

(11) **EP 3 254 627 A1**
(43) Date of publication of application: **13.12.2017**
(21) Application number: 16205160.1
(22) Date of filing: 19.12.2016
(51) Int. Cl.: A61B 6/12, A61B 17/34, A61B 6/00, A61B 10/00, A61B 90/13, A61B 90/00

(54) **FLUOROSCOPIC GUIDANCE SYSTEM WITH OFFSET LIGHT SOURCE AND METHOD OF USE**

(30) Priority: 08.06.2016 US 201615176751
(71) Applicant: General Electric Company, Schenectady, NY 12345 (US)
(72) Inventor: MCCARTHY, Thomas, 78533 Buc (FR)
(74) Representative: Bedford, Grant Richard

(57) **Abstract**

A system 10 and associated method is provided for image-guided navigation during a medical procedure. The system 10 includes a movable gantry 32 having an X-ray source 12, an X-ray detector 22 and a laser 50 disposed on the detector 22 offset from the area of the detector receiving the beam from the source 12, a movable table and a system controller 24 operably connected to the gantry 32, the X-ray source 12, the X-ray detector 22, the laser 50 and the table. In the method, the controller 24 determines an optimal trajectory for the insertion of an interventional device into the body to intersect the target, positions the gantry 32 to locate the laser 50 at a point where a laser beam is emitted from the laser 50 along the optimal trajectory outside of the X-ray beam, and takes additional images during the procedure to show the position of the interventional device in the body as compared with the optimal trajectory.

## Description

The subject matter disclosed herein relates to the field of image-guided interventional devices and methods, and more specifically to guiding devices and techniques utilized with the interventional devices and methods.

Interventional devices, procedures and methods are used in a variety of situations, such as providing the necessary treatment or diagnostic interaction with the tissue or region of interest within the patient but in a minimally invasive manner, thereby greatly lessening the trauma and recovery time for the patient undergoing the procedure or treatment. As a result, these minimally invasive medical interventional devices, procedures and methods are becoming increasingly important in the treatment of various conditions, including coronary heart disease and are also increasing in the field of biopsies, spinal column treatments and tumor ablations.

In a minimally invasive interventional procedure, one or more medical devices, e.g., a needle, are introduced into the body of a patient for treatment or diagnostic purposes. After the initial insertion of the needle into the patient's body, at least the tip of the needle is no longer visible for a physician performing the interventional procedure. In order to navigate the needle within the body of the patient, the needle must therefore be visualized in a suitable way to illustrate the position of the needle to enable the clinician to move the needle to the desired area of interest within the patient.

Various systems and methods are available today for determining the position of the instrument in the patient's body during minimally invasive medical interventions, which is necessary for visualizing the instrument, in particular the tip of the instrument, in image information from inside the patient's body. Some examples of these types of systems and methods are illustrated in US Patent No. 6,487,431 entitled *Radiographic Apparatus And Method For Monitoring The Path Of A Thrush Needle* and US Patent Application Publication No. US2008/0200876 entitled *Needle Guidance With Dual-Headed* Laser, both of which are expressly incorporated by reference herein in their entirety for all purposes. These systems and methods involve the use of image information obtained on the body region acquired preoperatively or intraoperatively by an imaging system, such as a computed tomography scanner, a magnetic resonance device or a C-arm X-ray device as 2D images, or as a 3D image. The information provided by the images can be utilized to assist the clinician in determining an appropriate insertion path along which to insert the interventional device into the patient. The initial point of the insertion path on the skin of the patient can be illuminated by a laser or other suitable light emitting device to enable the clinician to insert the device at the proper starting point. The imaging system can then obtain addition image information on the positioning of the device relative to the area of interest to guide the device.

However, certain significant issues are present with regard to these prior art systems and methods relates to the laser utilized to illuminate the insertion point on the skin of the patient. For example, in those systems where the laser is aligned coaxially with the imaging system, i.e., in a bull's-eye view where the laser and the X-ray beam emitted by the imaging system to strike the detector are coaxial, depending upon the position of the body of the patient, the area of interest within the patient, and the desired path of insertion, it may not be possible to position the imaging system at the proper location to enable the laser to illuminate the entry point on the patient.

A further drawback of the coaxial positon of the laser and the X-ray beam is that, because the point identified by the laser is positioned within the imaging field defined by the imaging system, the clinician must place his or her hands within the field in order to properly locate the interventional device on the patient. Doing so necessarily exposes the clinician to the radiation from the imaging system which is detrimental to the clinician, unless the clinician is utilizing an extender for the device, which necessarily limits the effectiveness of the positioning of the device by the clinician.

To address this issue, other prior art systems have been developed in which the laser is disposed on an armature separate from the imaging system, such as on a robotic arm. The arm can be positioned in order to illuminate the entry point on the patient for the interventional device, without need to be coupled to the imaging system. However, in these systems the clinician has to assume that the armature has properly positioned the laser with regard to the patient to illustrate the entry path, which is often not the case due to errors with regard to the operation of the system.

There is a need or desire for a system and method that can provide images for the guidance of an interventional device used in a minimally invasive surgical procedure that does not include the above-mentioned drawbacks and needs in the prior art. These issues are addressed by the embodiments described herein in the following description of the invention, which is a system and method for increasing the angular positions for the detector in which the procedure can be performed and without the need for the clinician/physician performing the procedure to place their hands within the beam striking the detector.

In the exemplary embodiments of the system and associated method, an imaging system utilized to obtain X-ray/fluoro images of the area of interest and the position of the interventional device within the patient includes one or more light sources or emitters, e.g., lasers, positioned on the detector for the imaging system. The lasers are positioned in multiple different offset locations with regard to the detector, such that the lasers are not coaxial with the X-ray beam. This positioning enables each laser to direct a laser beam at a location on the patient within the X-ray beam generated by the imaging system that enables the physician to access the point indicated by the laser beam with a tool without obstructing the view of the positioning laser and without the physician having to place their hands within the field of the X-ray beam. Further, the offset location of the laser enables the detector to be positioned at locations relative to the patient where the laser can project an entry point for the tool on the patient that were previously not possible with prior art coaxial systems. In addition, the system and method allows the clinician/physician to position the tool while simultaneously using the imaging system to check the position/depth of the interventional device as it is moved towards the area of interest.

According to one exemplary embodiment of the invention, a system for image-guided navigation of a tool is provided that includes an X-ray source capable of emitting an X-ray beam, an X-ray collimator capable of limiting the beam extent, an X-ray detector capable of detecting the X-ray beam on a first position of the detector and a light emitting device disposed on the detector in a second position, wherein the second positon is offset from the first position.

According to another exemplary embodiment of the invention, a method of providing image-guided navigation during a medical procedure includes the steps of providing an image-guided navigation system including a movable gantry on which is disposed an X-ray source capable of emitting an X-ray beam, an X-ray detector capable of detecting the X-ray beam on a first position of the detector and a light source disposed on the detector in a second position, wherein the second positon is offset from the first position, a target support disposed within the field of movement of the gantry and a system controller including a user input operably connected to the gantry, the X-ray source, the X-ray detector, the laser and the target support, operating the X-ray source and X-ray detector to obtain at least one image of a target within a body, determining an optimal trajectory for the insertion of a tool into the body to intersect the target, positioning the gantry to locate the light source at a point where a light beam is emitted from the light source along the optimal trajectory outside of the X-ray beam.

According to another exemplary embodiment of the invention, a method of providing image-guided navigation of a tool includes the steps of providing an image-guided navigation system including a movable gantry on which is disposed an X-ray source capable of emitting an X-ray beam, an X-ray detector capable of detecting the X-ray beam on a first position of the detector and a laser disposed on the detector in a second position, wherein the second positon is offset from the first position, a target support disposed within the field of movement of the gantry and a system controller including a user input operably connected to the gantry, the X-ray source, the X-ray detector, the laser and the table, operating the X-ray source and X-ray detector to obtain at least one image of a target within a body, determining an optimal trajectory for the insertion of a tool into the body to intersect the target, positioning the gantry to locate the laser at a point where a laser beam is emitted from the laser along the optimal trajectory, marking a calculated entry point on the body with the laser beam along the optimal trajectory, positioning a tip of the tool at the entry point, aligning the tool with the laser beam marking the optimal trajectory, inserting the tool into the body at the entry point along the optimal trajectory and obtaining an image of a position of the tool and the target within the body to compare with the optimal trajectory.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure

The drawings illustrate the best mode presently contemplated of carrying out the disclosure. In the drawings:
FIG. 1 is a schematic view of an X-ray/fluoro imaging and navigation system according to an exemplary embodiment of the invention.
FIG. 2 is a flowchart of a method of operation of an X-ray/fluoro imaging and navigation system according to an exemplary embodiment of the invention.
FIG. 3 is a schematic view of the operational capability of an X-ray/fluoro imaging and navigation system according to another exemplary embodiment of the invention compared with a prior art X-ray/fluoro imaging and navigation system.
FIG. 4 is a schematic view of an X-ray/fluoro imaging and navigation system according to another exemplary embodiment of the invention.

In the following detailed description, reference is made to the accompanying drawings that form a part hereof, and in which is shown by way of illustration specific embodiments, which may be practiced. These embodiments are described in sufficient detail to enable those skilled in the art to practice the embodiments, and it is to be understood that other embodiments may be utilized and that logical, mechanical, electrical and other changes may be made without departing from the scope of the embodiments. The following detailed description is, therefore, not to be taken in a limiting sense.

Exemplary embodiments of the invention disclosed herein relate to a system and method for identifying an optimal trajectory for the insertion of an interventional device into a patient and displaying the position of the device as it is inserted into the body of the patient towards an area of interest.

The system and method disclosed herein may be suitable for use with a range of imaging and navigation systems. To facilitate explanation, the present disclosure will primarily discuss the invention in the context of a C-arm fluoroscopic system. However, it should be understood that the following discussion may also be applicable to other and navigation systems.

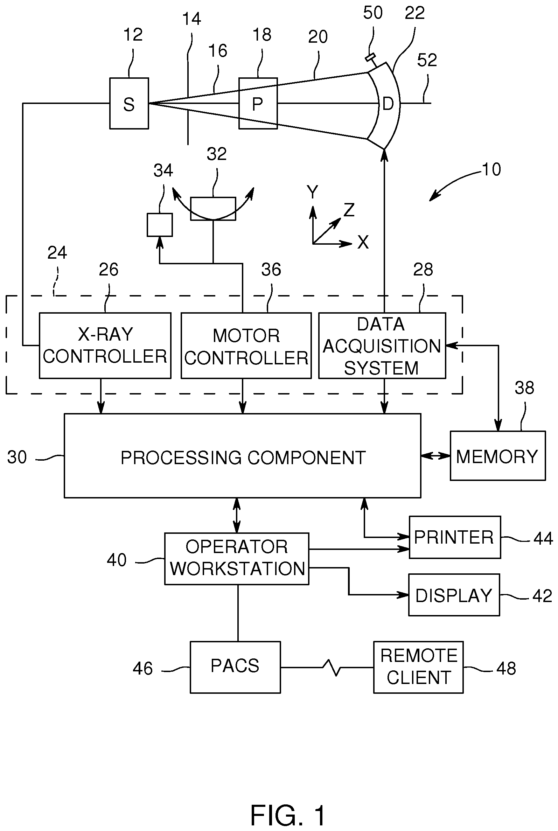

With this in mind, an example of a C-arm fluoroscopic imaging and navigation system 10 designed to acquire X-ray attenuation data at a variety of views around a patient and suitable for tomographic reconstruction is provided in FIG. 1. In the embodiment illustrated in FIG. 1, imaging system 10, such as that disclosed in US Patent No. 9,076,255, incorporated herein by reference in its entirety for all purposes, includes a source of X-ray radiation 12 positioned adjacent to a collimator 14. The X-ray source 12 may be an X-ray tube, a distributed X-ray source (such as a solid-state or thermionic X-ray source) or any other source of X-ray radiation suitable for the acquisition of medical or other images.

The collimator 14 permits X-rays 16 to pass into a region in which a patient 18, is positioned. In the depicted example, the X-rays 16 are collimated to be a cone-shaped or pyramidal-shaped beam, i.e., a cone-beam, that passes through the imaged volume of the patient 18 containing the area of interest or target T. A portion of the X-ray radiation beam 20 passes through or around the patient 18 (or other subject of interest) and impacts a detector array, represented generally at reference numeral 22. Detector elements of the array produce electrical signals that represent the intensity of the incident X-rays 20. These signals are acquired and processed to reconstruct images of the features within the patient 18.

Source 12 is controlled by a system controller 24, which furnishes both power, and control signals for the examination sequences. In the depicted embodiment, the system controller 24 controls the source 12 via an X-ray controller 26 which may be a component of the system controller 24. In such an embodiment, the X-ray controller 26 may be configured to provide power and timing signals to the X-ray source 12.

Moreover, the detector 22 is coupled to the system controller 24, which controls acquisition of the signals generated in the detector 22. In the depicted embodiment, the system controller 24 acquires the signals generated by the detector using a data acquisition system 28. The data acquisition system 28 receives data collected by readout electronics of the detector 22. The data acquisition system 28 may receive sampled analog signals from the detector 22 and convert the data to digital signals for subsequent processing by a processor 30 discussed below. Alternatively, in other embodiments the digital-to-analog conversion may be performed by circuitry provided on the detector 22 itself. The system controller 24 may also execute various signal processing and filtration functions with regard to the acquired image signals, such as for initial adjustment of dynamic ranges, interleaving of digital image data, and so forth.

In the embodiment illustrated in FIG. 1, system controller 24 is coupled to a linear and rotational subsystem 32 and a linear and rotational positioning subsystem 34. The rotational subsystem 32 enables the X-ray source 12, collimator 14 and the detector 22 to be rotated one or multiple turns around the patient 18, such as rotated primarily in an x, y-plane, or angulated with respect to the patient. The distance between the detector 22 and the X-ray source 12 can also be adjusted. It should be noted that the rotational subsystem 32 might include a gantry upon which the respective X-ray emission and detection components are disposed. Thus, in such an embodiment, the system controller 24 may be utilized to operate the gantry.

The linear and rotational positioning subsystem 34 may enable the patient 18, or more specifically a table supporting the patient, also called the target support, to be displaced within the imaging field of view of the system 10. Thus, the table may be linearly or rotationally moved (in a continuous or step-wise fashion) within the gantry to generate images of particular areas of interest or target T within the patient 18. In the depicted embodiment, the system controller 24 controls the movement of the gantry 32 and/or the positioning of the table 34 via a motor controller 36.

In general, system controller 24 commands operation of the imaging system 10 (such as via the operation of the source 12, detector 22, and positioning systems described above) to execute examination protocols and to process acquired data. For example, the system controller 24, via the systems and controllers noted above, may rotate a gantry supporting the source 12 and detector 22 about an area of interest or target T so that X-ray attenuation data may be obtained at a variety of views relative to the target T. In the present context, system controller 24 may also include signal processing circuitry, associated memory circuitry for storing programs and routines executed by the computer (such as routines for executing image processing techniques described herein), as well as configuration parameters, image data, and so forth.

In the depicted embodiment, the image signals acquired and processed by the system controller 24 are provided to a processing component 30 for reconstruction of images. The processing component 30 may be one or more conventional microprocessors. The data collected by the data acquisition system 28 may be transmitted to the processing component 30 directly or after storage in a memory 38. Any type of memory suitable for storing data might be utilized by such an exemplary system 10. For example, the memory 38 may include one or more optical, magnetic, and/or solid state memory storage structures. Moreover, the memory 38 may be located at the acquisition system site and/or may include remote storage devices for storing data, processing parameters, and/or routines for image reconstruction, as described below.

The processing component 30 may be configured to receive commands and scanning parameters from an operator via an operator workstation 40, typically equipped with a keyboard and/or other input devices. An operator may control the system 10 via the operator workstation 40. Thus, the operator may observe the reconstructed images and/or otherwise operate the system 10 using the operator workstation 40. For example, a display 42 coupled to the operator workstation 40 may be utilized to observe the reconstructed images and to control imaging. Additionally, the images may also be printed by a printer 44 which may be coupled to the operator workstation 40.

Further, the processing component 30 and operator workstation 40 may be coupled to other output devices, which may include standard or special purpose computer monitors and associated processing circuitry. One or more operator workstations 40 may be further linked in the system for outputting system parameters, requesting examinations, viewing images, and so forth. In general, displays, printers, workstations, and similar devices supplied within the system may be local to the data acquisition components, or may be remote from these components, such as elsewhere within an institution or hospital, or in an entirely different location, linked to the image acquisition system via one or more configurable networks, such as the Internet, virtual private networks, and so forth.

It should be further noted that the operator workstation 40 may also be coupled to a picture archiving and communications system (PACS) 46. PACS 46 may in turn be coupled to a remote client 48, radiology department information system (RIS), hospital information system (HIS) or to an internal or external network, so that others at different locations may gain access to the raw or processed image data.

While the preceding discussion has treated the various exemplary components of the imaging system 10 separately, these various components may be provided within a common platform or in interconnected platforms. For example, the processing component 30, memory 38, and operator workstation 40 may be provided collectively as a general or special purpose computer or workstation configured to operate in accordance with the aspects of the present disclosure. In such embodiments, the general or special purpose computer may be provided as a separate component with respect to the data acquisition components of the system 10 or may be provided in a common platform with such components. Likewise, the system controller 24 may be provided as part of such a computer or workstation or as part of a separate system dedicated to image acquisition.

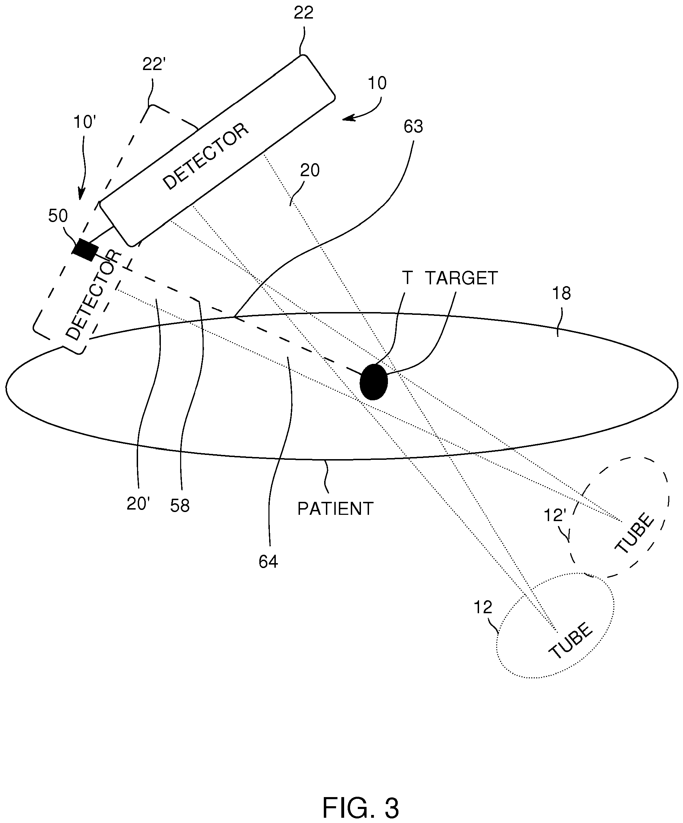

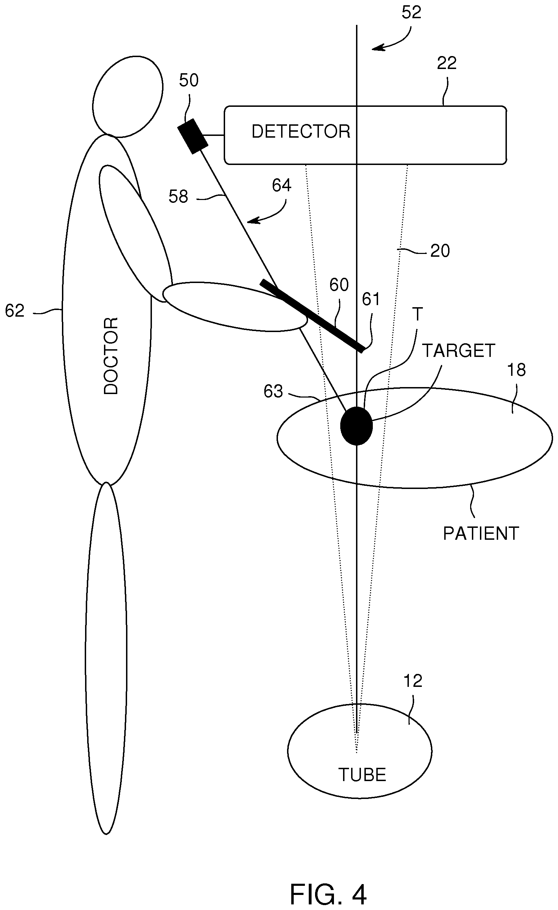

Referring now to FIG. 1 and 4, the system 10 additionally includes a light source or emitter, such as a laser 50, that is mounted to the detector 22 and operable by the system controller 24. The laser 50 can be secured to the detector 22 in any suitable manner such that the laser 50 is located in an offset position relative to the axis 52 of the X-ray beam 20 striking the detector 22. The laser 50 can be mounted directly to the detector 22, such as within a housing for the detector 22, or to the exterior of the detector 22 using a suitable support arm 56 or similar apparatus extending between the detector 22 and the laser 50, which may be collapsible or fold-away for storage within the detector 22 or that can be removable from the detector 22 by mechanical, magnetic or other suitable supporting devices. The laser 50 can include a battery (not shown) to power the laser 50. In another exemplary embodiment, the power, control and sensing signals used for the positioning and operation of the laser 50 can be provided by the system controller 24 as a feature of the attachment of the laser 50 to the detector 22. Further, while one laser 50 is shown in the exemplary embodiments of FIGS. 1, 3 and 4, multiple lasers 50 can be mounted to the detector 22 at different positions and angular orientations relative to the detector 22. Each additional laser 50 can extend the angular reach of the system 10, allowing it to align at least one laser 50 with the desired needle path when the bull's eye view is not reachable, such as shown in FIG. 3. In the case where the desired needle path can be aligned with multiple lasers 50 without patient-gantry interference, the system controller 24 decides which laser 50 to turn on and align with the optimized trajectory 64 using computation and the clinical task information provided by the user. In this position the laser 50 can project a laser beam 58 onto the patient 18 that corresponds to the entry point 63 and trajectory 64 calculated for the particular tool or interventional device 60 to be utilized, such as in a medical procedure. The physician 62 performing the procedure can then place the interventional device tip 61 at the entry point 63 then the interventional device 60 along the trajectory 64 indicated by the laser beam 58 in order to insert the device 60 into the patient 18.

Each laser 50 is mounted to the detector 22 at a static and known position, such that the system 10 includes a value for the offset angle and distance that the laser 50 is positioned from the center of the X-ray beam 20 striking the detector 22. However, in another exemplary embodiment, the laser(s) 50 can be movably positionable relative to the detector 22. In this exemplary embodiment, once the laser(s) 50 is in the desired position, the system 10 determines the location (e.g., the offset angle and distance) of each laser 50 relative to the detector 22 for use in the procedure to be performed utilizing the system 10.

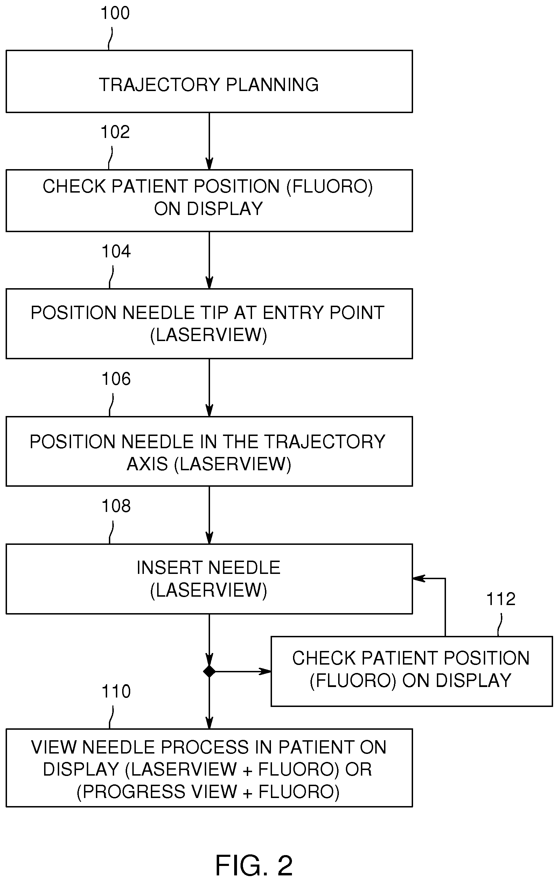

Looking at FIG. 2, an exemplary embodiment of the method of operation of the imaging/navigation system 10 to perform a procedure and track the device 60 is depicted in flowchart form. As depicted at block 100, initially the system controller 24 computes and plans the trajectory 64 for the insertion of the interventional device 60 including the entry point 63 and the angle of the device 60 in one or more planes relative to the entry point 63. This determination can be performed by imaging the target T within the patient 18 using the system 10 and using the images obtained in a suitable program, such as the Innova TrackVision software package available from GE Healthcare and disclosed in US Patent No. 8,600,138 entitled *Method for processing radiological images to determine a 3D position of a needle,* which is expressly incorporated herein by reference in its entirety for all purposes, to create a suitable 3D image of the target T and surrounding tissues in the patient 18 and determine/compute the optimal trajectory 64 for reaching the target T from the exterior of the patient 18.

Once the optimal trajectory 64 is determined, in block 102 the system controller 24 determines the gantry 32 and patient support 34 position for the Bull's Eye view as well as for the "Laserview" and a Progress view used by the system 10. The Laserview is defined by the location of the detector 22 relative to the patient 18 where the selected laser 50 is positioned to illuminate the entry point 63 by directing the laser beam 58 along the computed optimal trajectory 64 to the target T, as shown in FIG. 4. The laser beam can be a simple line projecting a dot or in the shape of a cross-hair. In the Bull's-Eye view, the detector 22 and source 12 are aligned to project the X-ray beam 20 along the trajectory 64. Ina Progress view, the detector 22 and source 12 are oriented to project the X-ray beam 20 at a ninety degree (90°) angle relative to the trajectory 64. As there are multiple Progress views, which is any view for the X-ray beam 20 that is oriented perpendicular to the needle trajectory 64, the system controller 24 selects one Progress view which can be reached while minimizing the likelihood of a collision between the detector 20 and the table/patient support 34 and while also minimizing the distance between the Laserview position and the selected Progress view position, thereby preventing excessive travel for the system 10. The Laserview may not be coplanar with the Bull's eye view and/or the selected Progress view.

The Laserview position is achieved by adjusting the gantry 32 and patient support/table 34 linearly and/or rotationally to align the laser beam 58 with the desired angles for the trajectory 64. The position of each angle/axis is determined by the system controller 24 using the trajectory (α,β) determined at step 100 offset by the known angle/position of the laser 50 relative to the detector 22. In one exemplary embodiment, the gantry 32 and table 34 provide up to 5 extra degrees of freedom (2 rotations and 3 translations) enabling multiple solutions for the Laserview position to be determined, in which case, the system controller 24 selects an optimal position given the clinical task/procedure to be performed as indicated to the system 10 by the operator. The Laserview can be reached by the movement of the gantry 32 alone, provided that it has 2 rotational and 3 translational degrees of freedom such as the Discovery IGS 740 from GE Healthcare. However, many implementations will have the gantry 32 provide the 2 rotational degrees of freedom and the patient support/table 34 provide the 3 translational degrees of freedom.

In determining the Laserview configuration for the source 12 and detector 22, the system controller 24 additionally determines which laser 50 should be aligned to the trajectory 64 based on whether the position for the detector 22 at that trajectory 64 is reachable or not due to potential interference/collisions, for example between the detector 22 and/or tube 12 and the patient 18 and/or table 34 on which the patient is positioned and in order to optimize the access of the physician 62 at that trajectory 64, as shown in FIG. 3 in comparison with a prior art system 10' including a source 12', an X-ray beam 20' emitted from the source 12' and a detector 22'. As illustrated, the system 10 including the laser 50 allows the detector 22 to be used to reach targets T using paths which would not have been possible with prior art systems 10'. Further, with more lasers 50 disposed on the detector 22, the span of reachable angles or trajectories 64 is increased. Further, the step in block 100 can be performed completely automatically by the system controller 24, by inputs from the physician 62 to the system controller 24, or a combination thereof.

After the Laserview to be utilized in the procedure has been determined, in block 102 the system controller 24 can optionally operate rotational subsystem 26 to position the source 12 and the detector 22 in a Bull's-eye view configuration relative to the trajectory 64 where the target T is centered in the image, if this view is reachable. At this position one or more images are taken at this position to verify the location of the target T and that the patient 18 has not moved thereby confirming the trajectory 64. If the Bull's eye view is not reachable, other suitable views such as two orthogonal views can be used.

Once the positon of the target T and patient 18 has been confirmed, the method proceeds to block 104 where the system controller 24 moves the source 12 and detector 22 back to the Laserview configuration where the physician places the tip 61 of the interventional device/needle 60 at the entry point 63 indicated by the beam 58 emitted from the laser 50.

In block 106 the physician then moves the device 60 in order to align the device 60 with the trajectory 64 indicated by the angles of the beam 58 relative to the entry point 63, while keeping the tip 61 on the entry point 63, schematically shown in FIG. 4. In block 108, once the device 60 has been positioned at the entry point 63 along the planned trajectory 64, the physician 62 then inserts the device 60 into the body of the patient 18. These steps can be readily accomplished as shown in FIG. 3 where the tip of the device 60 is placed at the entry point 63 while the hands of the physician 62 remain outside of the X-ray beam 20. In addition, the laser beam 58 can be readily and easily viewed due to the unobstructed view of the trajectory 64 indicated by the laser beam 58 based on the offset position of the laser 50.

As the physician is inserting the device 60 into the patient 18, upon the request of the physician, in block 110 the system controller 24 can position the gantry 32 and/or table 34 at the Laserview or Progress view. In doing so, the system controller 24 can take one or more additional images of the patient 18 in order to display the position of the tip of the device 60 relative to the trajectory 64 and to the target T. In the Laserview configuration, with the only assumption being that the device 60 is inserted at the entry point 63 and aligned with the laser beam 58, the system controller 24 can readily illustrate to the physician the three-dimensional position and depth of the device 60 along the trajectory 64 simultaneously with the physician holing the device 60 to assist in guiding the device 60 to the target T. This can be shown to the physician on the display 42, optionally with the image taken in the Laserview position used as an overlay over a three-dimensional image of the planned trajectory through the patient 18 compiled from prior images. From this combined image, the system controller 24 can indicate to the physician the exact position of the device 60 in the patient 18, accuracy of the insertion of the device 60 along the trajectory 64, can identify any corrections that need to be made to the device 60 placement, and can display the remaining distance between the device 60 and the target T, among other suitable information concerning the procedure.

Alternatively, where the image is obtained in a Progress view configuration for the source 12 and detector 22, the system controller 24 moves the gantry 32 to the appropriate position to obtain the Progress view image, and then optionally returns the gantry 32 to the Laserview position. The Progress view image can then be used by itself or with the three-dimensional trajectory planning image to enable the physician to see the device 60 as it is being manipulated and its position in the patient 18 relative to the planned trajectory 64. Also, switching between these view configurations can provide access to more information at a lower dose while maintaining the accuracy.

After insertion of the device 60 in block 110, or as another check prior to the insertion of the device 60, the physician can request that the system controller 24 perform a check on the position of the patient 18 in order to determine that the patient 18 is still in the proper position relative to the planned trajectory 64. To do so, in block 112, upon the request of the physician, the system controller 24 operated the gantry 32 and/or or the table 34 to place the source 12 and detector 22 in one or more suitable views, potentially including the Bull's-eye view configuration. The system controller 24 then operates the source 12 and detector 22 to obtain an image is taken of the target T and check that the target T is at the proper location relative to the planned trajectory 64.

The written description uses examples to disclose the invention, including the preferred mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

Various aspects and embodiments of the present invention are defined by the following numbered clauses:
1. A system for image-guided navigation of tools, the system comprising:
   an X-ray source capable of emitting an X-ray beam;
   an X-ray detector capable of detecting the X-ray beam on a first position of the detector; and
   a light emitting device disposed on the detector in a second position, wherein the second positon is offset from the first position.
2. The system of clause 1, wherein the light emitting device is disposed within a housing for the detector.
3. The system of any preceding clause, wherein the light emitting device is secured to an exterior of a housing for the detector.
4. The system of any preceding clause, wherein the light emitting device is releasably attached to the exterior of the detector.
5. The system of any preceding clause, wherein the light emitting device is movable with respect to the detector.
6. The system of any preceding clause, further comprising a plurality of light emitting devices disposed at positions offset from the first position of the detector.
7. A method of providing image-guided navigation of a tool, the method comprising the steps of:
   providing an image-guided navigation system including a movable gantry on which is disposed an X-ray source capable of emitting an X-ray beam, an X-ray detector capable of detecting the X-ray beam on a first position of the detector and a light source disposed on the detector in a second position, wherein the second positon is offset from the first position, a target support disposed within the field of movement of the gantry and a system controller including a user input operably connected to the gantry, the X-ray source, the X-ray detector, the light source and the table;
   operating the X-ray source and X-ray detector to obtain at least one image of a target within a body;
   determining an optimal trajectory for the insertion of a tool into the body to intersect the target; and
   positioning the gantry to locate the light source at a point where a light beam is emitted from the light source along the optimal trajectory outside of the X-ray beam.
8. The method of any preceding clause, wherein the step of positioning the gantry locates the detector at a location offset from the optimal trajectory.
9. The method of any preceding clause, further comprising the steps of:
   calculating an entry point on the body for the optimal trajectory along with calculating the optimal trajectory; and
   marking the entry point on the body with the light beam along the optimal trajectory.
10. The method of any preceding clause, further comprising the steps of:
   positioning a tip of the tool at the entry point;
   aligning the tool with the light beam marking the optimal trajectory; and
   inserting the tool into the body at the entry point along the optimal trajectory.
11. The method of any preceding clause, further comprising the step of obtaining an image of a position of the tool and the target within the body after inserting the tool into the body.
12. The method of any preceding clause, wherein the step of obtaining the image is simultaneous with inserting the tool into the body along the optimal trajectory.
13. The method of any preceding clause, wherein the step of obtaining an image comprises operating the X-ray source and the X-ray detector to obtain an image of the target and the tool where the X-ray beam emitted from the X-ray source is offset from the optimal trajectory.
14. The method of any preceding clause, wherein the step of obtaining an image further comprises operating the X-ray source and the X-ray detector to obtain another image of the target and the tool where the X-ray beam emitted from the X-ray source is emitted along the optimal trajectory.
15. The method of any preceding clause, wherein the step of obtaining an image comprises operating the X-ray source and the X-ray detector to obtain an image of the target and the tool where the X-ray beam emitted from the X-ray source is offset at an angle of ninety degrees (90°) from the optimal trajectory.
16. The method of any preceding clause, further comprising the step of displaying the image of the target and the tool.
17. The method of any preceding clause, wherein the step of displaying the image of the target and the tool comprises displaying the image overlaying a representation of the optimal trajectory through the body to the target.
18. The method of any preceding clause, wherein the step of determining the optimal trajectory comprises selecting one of a plurality of light sources secured to the detector capable of emitting a light beam along the optimal trajectory without interference between the detector and the body or table.
19. A method of providing image-guided navigation of a tool, the method comprising the steps of:
   providing an image-guided navigation system including a movable gantry on which is disposed an X-ray source capable of emitting an X-ray beam, an X-ray detector capable of detecting the X-ray beam on a first position of the detector and a laser disposed on the detector in a second position, wherein the second positon is offset from the first position, a target support disposed within the field of movement of the gantry and a system controller including a user input operably connected to the gantry, the X-ray source, the X-ray detector, the laser and the table;
   operating the X-ray source and X-ray detector to obtain at least one image of a target within a body;
   determining an optimal trajectory for the insertion of a tool into the body to intersect the target;
   positioning the gantry to locate the laser at a point where a laser beam is emitted from the laser along the optimal trajectory;
   marking a calculated entry point on the body with the laser beam along the optimal trajectory;
   positioning a tip of the tool at the entry point;
   aligning the tool with the laser beam marking the optimal trajectory;
   inserting the tool into the body at the entry point along the optimal trajectory; and
   obtaining an image of a position of the tool and the target within the body to compare with the optimal trajectory.

## Claims

1. A system (10) for image-guided navigation of tools, the system comprising:
an X-ray source (12) capable of emitting an X-ray beam;
an X-ray detector (22) capable of detecting the X-ray beam on a first position of the detector; and
a light emitting device (50) disposed on the detector in a second position, wherein the second positon is offset from the first position.

2. The system (10) of claim 1, wherein the light emitting device (50) is disposed within a housing for the detector (22).

3. The system (10) of any preceding claim, wherein the light emitting device (50) is secured to an exterior of a housing for the detector (22).

4. The system (10) of claim 3, wherein the light emitting device (50) is releasably attached to the exterior of the detector (22).

5. The system (10) of any preceding claim, wherein the light emitting device (50) is movable with respect to the detector (22).

6. The system (10) of any preceding claim, further comprising a plurality of light emitting devices disposed at positions offset from the first position of the detector (22).

7. A method of providing image-guided navigation of a tool, the method comprising the steps of:
providing an image-guided navigation system (10) including a movable gantry (32) on which is disposed an X-ray source (12) capable of emitting an X-ray beam, an X-ray detector (22) capable of detecting the X-ray beam on a first position of the detector and a light source (50) disposed on the detector (22) in a second position, wherein the second positon is offset from the first position, a target support disposed within the field of movement of the gantry (32) and a system controller (24) including a user input operably connected to the gantry, the X-ray source, the X-ray detector, the light source and the table;
operating the X-ray source (12) and X-ray detector (22) to obtain at least one image of a target within a body;
determining an optimal trajectory for the insertion of a tool into the body to intersect the target; and
positioning the gantry (32) to locate the light source (50) at a point where a light beam is emitted from the light source along the optimal trajectory outside of the X-ray beam.

8. The method of claim 7, wherein the step of positioning the gantry (32) locates the detector (22) at a location offset from the optimal trajectory.

9. The method of claim 7 or claim 8, further comprising the steps of:
calculating an entry point on the body for the optimal trajectory along with calculating the optimal trajectory; and
marking the entry point on the body with the light beam along the optimal trajectory.

10. The method of any of claims 7 to 9, further comprising the steps of:
positioning a tip of the tool at the entry point;
aligning the tool with the light beam marking the optimal trajectory; and
inserting the tool into the body at the entry point along the optimal trajectory.

11. The method of any of claims 7 to 10, further comprising the step of obtaining an image of a position of the tool and the target within the body after inserting the tool into the body.

12. The method of any of claims 7 to 11, wherein the step of obtaining the image is simultaneous with inserting the tool into the body along the optimal trajectory.

13. The method of any of claims 7 to 12, wherein the step of obtaining an image comprises operating the X-ray source (12) and the X-ray detector (22) to obtain an image of the target and the tool where the X-ray beam emitted from the X-ray source is offset from the optimal trajectory.

14. The method of any of claims 7 to 13, wherein the step of obtaining an image further comprises operating the X-ray source (12) and the X-ray detector (22) to obtain another image of the target and the tool where the X-ray beam emitted from the X-ray source is emitted along the optimal trajectory.

15. The method of any of claims 7 to 14, wherein the step of obtaining an image comprises operating the X-ray source (12) and the X-ray detector (22) to obtain an image of the target and the tool where the X-ray beam emitted from the X-ray source is offset at an angle of ninety degrees (90°) from the optimal trajectory.
